# EUROPEAN PATENT APPLICATION

(11) **EP 2 377 459 A1**
(43) Date of publication of application: **19.10.2011**
(21) Application number: 09832184.7
(22) Date of filing: 23.09.2009
(51) Int. Cl.: A61B 5/00, A61B 5/02, G01H 1/00, G01L 7/18

(54) **METHOD FOR MEASURING ARTERIAL PRESSURE AND A DEVICE FOR THE IMPLEMENTATION OF SAME**

(30) Priority: 12.12.2008 RU 2008148919
(71) Applicant: Romanovski, Vladimir Fedorovich, Moscow 127642 (RU); Romanovski, Aleksei Vladimirovich, Moscow 124365 (RU); Litvinov, Aleksandr Vasilievich, Korolev 141070 (RU)
(72) Inventor: ROGOZA, Anatoli Nikoleavich, Moscow 125362 (RU); ROMANOVSKAYA, Antonina Mikhailovna, Moscow 127642 (RU)
(74) Representative: Tollett, Ian
(86) International application number: PCT/RU2009/000491
(87) International publication number: WO 2010/068135

(57) **Abstract**

A method for measuring arterial pressure involves hydraulically separating a static pressure source from a body area situated opposite thereto and containing an artery by placing a rigid separating element between the two and along the artery. With the aid of the separating element, pulse waves are converted into pressure waves, which run along said element on the first side thereof, and the passage of the pulse waves is detected with the aid of the pressure waves. A device for measuring arterial pressure comprises a rigid separating element which has a first contact surface for interacting with the body and a second contact surface which is opposite to the first and is used for interacting with the static pressure source. A push-type pressure gauge with a contact area that is embedded into one of the two contact surfaces of the separating element and a pulse wave detector are disposed inside the rigid separating element. The sensing element of the detector is located on the first contact surface.

## Description

### TECHNICAL FIELD

The present invention relates to medical technology, namely to cardiologic diagnostics and to devices for hemodynamic research, particularly, to a method for measuring arterial pressure and a device for implementation of the same.

### PRIOR ART

Known in the art are numerous methods and devices for measuring arterial pressure, which are used both in clinical practice and domiciliary self-diagnostics.

Thus, RU 895405 discloses a method and device for indirect measuring arterial pressure.

This prior art method for indirect measuring arterial pressure comprises applying external pressure to a body area and clamping the artery passing through the same, varying the value of external pressure within the range covering the values of systolic and diastolic pressure, recording the pulse waves of blood at a more distal position relative to the site of clamping the artery, measuring external pressure at a time of onset and disappearance of the pulse waves, said recording of the pulse waves being carried out based on the angular oscillations of the body surface relative to the direction of travel of said waves.

The device implementing this prior art method is configured as a pulse wave sensor comprising a housing, a bandage with a contact surface and a converter for converting the bandage oscillations into an electrical signal, said converter being rigidly fixed within the housing and mechanically connected to the bandage hingedly mounted within the housing and capable of angular oscillations about the hinge axis parallel to the bandage contact surface, the projection of which axis on the bandage contact surface coincides with the axis of symmetry of said surface.

The prior art method has a drawback consisting in that the accuracy of measuring arterial pressure is influenced by individual properties of the patient's body tissues since the pulse waves are recorded based on the angular oscillations, i.e. angular displacements of the body surface. The amplitude of such displacements depends on the elasticity of the body tissues, presence or absence of the subcutaneous adipose interlayer, elasticity of the material of which the cuff is made, etc., all that complicating safe recording of the pulse waves in each particular case of carrying out measurements.

The prior art device has a drawback consisting in that the amplitude of the bandage angular displacements relative to the hinge axis are converted into an electrical signal. However, since the bandage is pressed to the body surface, the amplitude of these displacements and the output electrical signal depend to a large extent on the properties of the patient's body tissues. In addition, the prior art device is adapted to convert into an electrical signal not just abstract oscillations of the bandage but its oscillations relative to the housing which itself arbitrary moves under the effect of these waves. All that complicates safe recording of the pulse waves of blood, affects negatively the accuracy of measuring arterial pressure and narrows the range of applicability of the device.

As the closest prior art, RU 2033746 entitled "Method for measuring arterial pressure and a device for the implementation the same" is selected.

A method for measuring arterial pressure is disclosed therein, said method comprising clamping an artery by means of a static pressure source (cuff), varying the cuff pressure to the body surface within the value range necessarily including possible values of arterial pressure, measuring the cuff pressure to the body surface, detecting passage of the pulse waves by means of a detector provided with a bandage, determining the values of systolic and diastolic arterial pressure based on the measured cuff pressure to the body surface at a time of onset and disappearance of the pulse waves, said detecting of the pulse waves being carried out along with recording the generated moment of forces acting upon the bandage as the pulse waves travel under the detector.

The device implementing the prior art method comprises a static pressure source (cuff) with a system for measuring and recording the pressure, a pulse wave detector configured as a housing, a bandage with a contact surface, a system for converting the bandage state into an electrical signal, said bandage being fixed within the bandage state conversion system sensitive to variations in the moment of forces acting upon the bandage, and said bandage being configured as at least two piezoelectric converters arranged symmetrically relative to the axis extending through the geometric center of the bandage contact surface perpendicularly to said surface.

The drawback of the prior art method consists in that the detector placed under the cuff and provided with the bandage is unable to select reliably the pulse waves based on the moment of forces acting upon the bandage as the pulse waves travel under the detector. The pulse wave of blood which is generated within the artery causes the body tissues surrounding the artery to displace as the wave travels. The patient's body and the cuff placed thereon with the pulse wave detector comprise a non-rigid system which may change its form as the pulse wave of blood travels along the artery.

Therefore, displacement of the tissues surrounding the artery as the pulse wave travels along the same also causes the detector itself to displace. In addition, the body and the cuff exhibit virtually no resistance to such displacement so that no noticeable force can be generated on the bandage contact surface since, being fixed within the detector housing, the bandage also displaces with the detector. At the same time, the bandage takes up only the difference of forces acting upon the bandage contact surface and the remaining part of the detector, said forces being in turn defined only by the elasticity of the body tissues and the cuff material. Furthermore, the forces generating as a result of the patient's muscular activity superimpose on the forces generated by the travelling pulse wave of blood so that false signals or in other words interfering signals emerge. The experience of application of this prior art method shows that for the above-mentioned reasons its interference immunity is insufficient for providing the appropriate accuracy of arterial pressure measurement so that it does not enable safe measurement of arterial pressure when a patient is in motion. Moreover, the prior art does not take into consideration that the cuff pressure to the body surface is not completely identical to the real external pressure acting upon the artery, which also impairs the accuracy of arterial pressure measurement.

Another drawback of the prior art device is the lack of rigid attachment of the detector under the compression cuff, leading to attenuation of the amplitude of the moment of forces taken up by the bandage contact surface and to a wide spread of this amplitude. It impairs the accuracy of arterial pressure measurement, narrows the field of application of this prior art device and also complicates the measurement of arterial pressure when a patient is in motion. In addition, pressure measurement in the cuff by means of a pressure gauge conventionally attached to the cuff by means of a tube introduces certain additional error into arterial pressure measurement associated with the dynamic error defined by the pressure difference between the ends of this tube and with the existing difference between the cuff pressure and the body pressure. Furthermore, the traditional way of measuring the external static pressure diminishes the self-sufficiency of the arterial pressure measuring device itself relative to the cuff, which complicates the pressure measurement process as such.

### DISCLOSURE OF THE INVENTION

It is an object of the present invention to provide a method for measuring arterial pressure, which makes it possible to improve the accuracy of measurement, to enhance the method versatility for extending the field of its application, and to simplify the measurement process by a medical worker.

The above object is achieved by the inventive method for measuring arterial pressure, wherein a pressure is produced, by means of a static pressure source, within a body area containing an artery; the value of said static pressure is varied within a value range which necessarily includes a possible range of arterial pressure values; the passage of pulse waves is detected; the value of systolic and diastolic arterial pressure is determined based on said static pressure at a time of onset and disappearance of said pulse waves. According to a first aspect of the invention, said static pressure source is caused to hydraulically separate from a body area situated opposite thereto and containing an artery by placing a rigid separating element between the two and along the artery, said rigid separating element having a first side facing the body and a second side facing said static pressure source, said pulse waves are converted with the aid of said separating element into pressure waves which run along said rigid separating element on the first side thereof, and the passage of said pulse waves is detected with the aid of said pressure waves which run along the surface of said rigid separating element first side.

In a particular embodiment, the values of systolic and diastolic arterial pressure are determined based on the determined maximum and minimum values of said static pressure applied to the rigid separating element in the vicinity of the cuff center at a time of onset and disappearance of said pulse waves.

The above object is also achieved by means of a device for measuring arterial pressure, comprising a static pressure source, a means for varying and measuring static pressure, a pulse wave detector, as well as a means for processing output signals. According to a second aspect of the invention, said device comprises a rigid separating element which has a first contact surface for interacting with the body and a second contact surface arranged opposite thereto for interacting with said static pressure source, wherein inside said rigid separating element, said means for measuring static pressure is configured as a push-type pressure gauge with a contact area embedded into one of the two contact surfaces of said separating element, as well as said pulse wave detector whose sensing element is located on the first contact surface of said separating element.

Preferably, the second contact surface of said rigid separating element is provided with a limiter for locking the position of said element under said static pressure source.

In a particular embodiment, the static pressure source is configured as a cuff.

In a particular embodiment, said sensing element of said pulse wave detector comprises a bandage embedded into said first contact surface of said rigid separating element by means of a hinge arranged along said bandage line of symmetry extending transversely to an artery.

### BRIEF DESCRIPTION OF THE DRAWINGS

The invention will be further illustrated by particular exemplary embodiments thereof and the accompanying drawings in which:
Fig. 1 schematically shows a device for implementing the method for measuring arterial pressure according to a first aspect of the invention;
Fig. 2 shows a diagram of the pressure wave running along the rigid separating element on a first side thereof in the blood flow direction;
Fig. 3 shows a diagram of the real distribution of static pressure generated inside the body at the site of the artery under the action of the static pressure source;
Fig. 4 shows the cross-sectional view of the device illustrated in Fig.1;
Fig. 5 shows a particular exemplary embodiment of the device for implementing the inventive method;
Fig. 6 shows a portion of the device illustrated in Fig. 5.

### BEST MODE FOR CARRYING OUT THE INVENTION

In order to implement the inventive method for measuring arterial pressure and achieve the above objects and effects consisting in improving the accuracy of measurement, the static pressure source 1 is caused to hydraulically separate from a body 2 area containing an artery 3. In the example shown in the drawings, a standard cuff known in the prior art is used as the static pressure source 1. In the following description, the source 1 is for simplicity referred to as the cuff, however, it will be appreciated by a skilled person that other suitable means may also be used as the source 1.

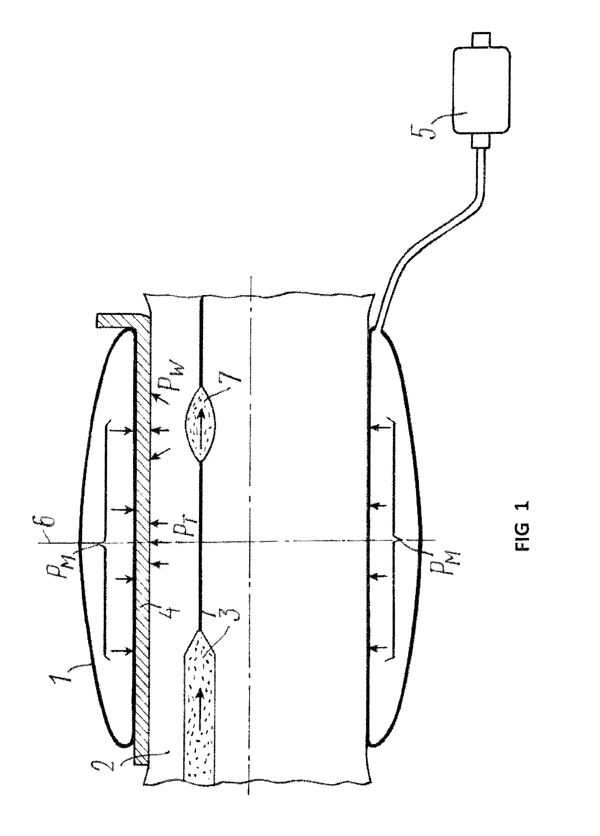

As shown in Fig. 1, to cause the cuff 1 to hydraulically separate from the body 2, a rigid separating element 4 is placed therebetween at the site of the artery 3 (and along the same across the cuff width), said separating element 4 having a first side facing the body 2 and a second side opposing said first side and facing the cuff 1. The rigid separating element 4 may, in particular, comprise a flat plate of a rigid material sized such as to be placed between the cuff 1 and an area of the patient's body 2.

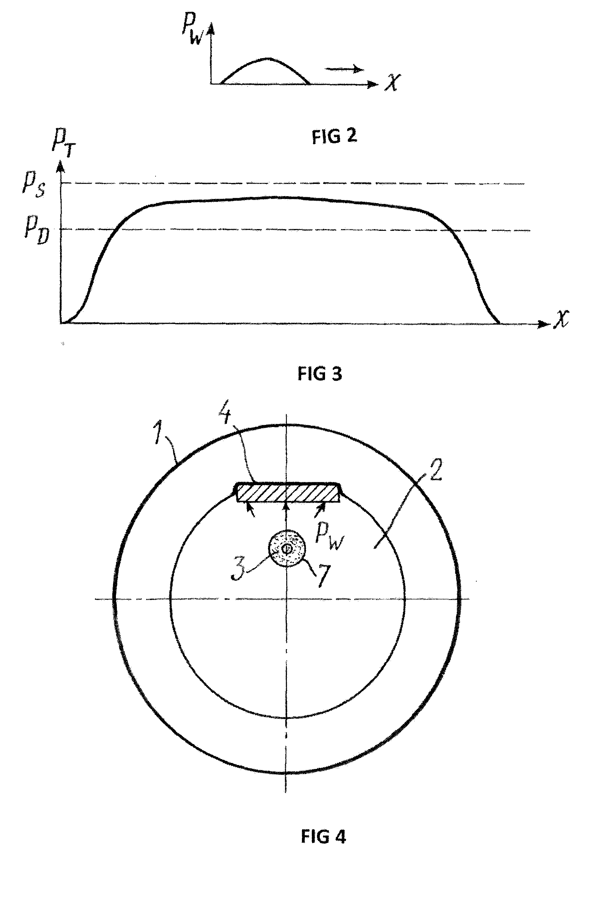

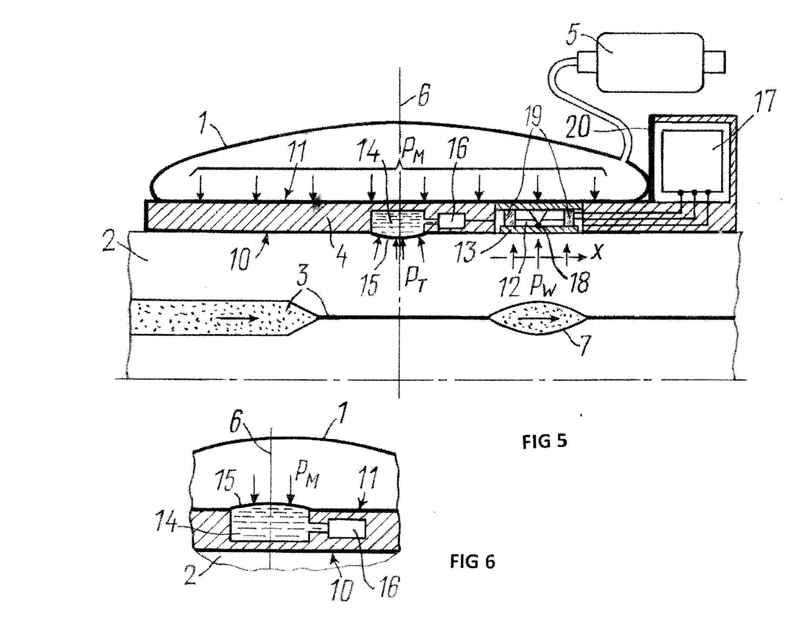

For measuring arterial pressure by means of the cuff 1, static pressure P_{M} is then produced to the surface of the body 2 area containing the artery 3 whereby static pressure P_{T} (Fig. 3) is formed within the body at the site of the artery under the action of pressure P_{M}, by means of a unit for varying the static pressure, the value of static pressure P_{T} is varied within the value range necessarily including possible values of arterial pressure from systolic pressure P_{S} to diastolic pressure P_{D}. The blood flow in the artery 3 has an intermittent nature within this value range of static pressure P_{T}. With each cardiac muscle contraction, the arterial pressure for a short time increases to the systolic value Ps. This being the case, the value of arterial pressure for a short time exceeds the static pressure P_{T} so that the artery 3 opens for this short time and lets a portion of blood pass under the cuff 1. Upon cardiac muscle relaxation, the arterial pressure goes down deeply to its diastolic value P_{D} so that the artery again closes under the action the exceeding external static pressure P_{T}. The value of static pressure is non-uniform along the X axis and reaches its peak in the vicinity of the cuff 1 center 6. Therefore, the artery 3 opening and closing processes start and stop in the vicinity of the cuff 1 center 6. The portions of blood having flown through the artery past the cuff 1 center 6 will be then forced out from under the cuff 1 in the blood flow direction to form the pulse waves 7 under its distal part, which run in the same direction and comprise thickenings of the artery 3 moving along the artery as shown in Figs. 1 and 5. The pulse waves 7 running along the artery 3 cause the body 2 tissues to move apart and form the waves of lateral displacement of these tissues adjacent to the artery 3. The rigid separating element 4 arranged between the artery 3 and the cuff 1 prevents the body 2 tissues from displacing beyond the rigid element 4 whereby pressure waves P_{W} (Fig. 2) are generated on its surface facing the body 2. Thus, as shown in Fig. 4, the pulse waves 7 are converted into the pressure waves P_{W} of the body 2 tissues to the surface of the rigid separating element 4 facing the body 2. The discussed mechanism for converting the pulse waves 7 into the pressure waves P_{W} makes it possible to detect clearly the pulse waves 7 by detecting the pressure waves P_{W} of the body 2 to the rigid separating element 4.

The static pressure P_{M} produced by the cuff 1 on the surface of the body 2 area is not identical in magnitude to the real static pressure P_{T} produced within the body 2 area under the cuff 1. The body 2 tissues have intrinsic elasticity. Upon compression of a body 2 area by means of the cuff, these tissues are deformed and partially displaced from the cuff 1 center 6 to its ends such that the resulting real static pressure P_{T} within the body 2 area at the site of the artery 3 becomes non-uniform with its peak value occurring in the vicinity of the cuff 1 center 6 and decreasing towards its ends, as shown in Fig. 3. While the real static pressure P_{T} within the body 2 at the site of the artery 3 is the closest to the value P_{M} in the vicinity of the cuff 1 center 6, it somewhat differs from the same. Therefore, instead of traditionally measuring static pressure P_{M}, static pressure P_{T} of the body 2 to the rigid separating element 4 in the vicinity of the cuff 1 center 6 may be measured to improve the accuracy of measuring arterial pressure.

The device for measuring arterial pressure in more detail shown in Fig. 5, comprises the cuff 1, the unit 5 for varying static pressure P_{M} configured as a traditional rubber blower for blowing air into the cuff 1, the rigid separating element 4 with two opposing contact surfaces, i.e. a first surface 10 and a second surface 11, said first surface 10 contacting the body 2 surface. Inside the rigid separating element 4 there is arranged a pulse wave detector 12 sensitive to the moment of forces acting on its bandage 13, and a static pressure P_{T} sensor 14 filled with a liquid and provided with a contact pad 15. The bandage 13 of the pulse wave detector 12 is embedded into the first contact surface 10 of the separating element 4. As shown in Fig. 6, the contact pad 15 of the static pressure sensor 14 is embedded into one of the two opposing contact surfaces 10 or 11, and the sensor 14 is hydraulically connected to a converter 16 for converting the hydraulic pressure into electrical signal. The separating element 4 may comprise a standard prior art unit 17 for processing the output signals into electrical signal, however the unit 17 may also be provided separately from the separating element 4. The bandage 13 of the pulse wave detector 12 is embedded into the first contact surface 10 of the element 4 by means of a hinge 18 arranged along the hinge centerline and is mechanically coupled with the separating element 4 by means of a piezoelectric converter 19 for converting the moment of forces into electrical signal. For convenience in operation and resettability of measurement results, the device may be provided with a limiter 20 for fixing the position of the separating element 4 under the cuff 1.

The proposed device operates as follows.

The cuff 1 is placed onto the body 2 area containing the artery 3. The separating element 4 is pushed in from the cuff 1 distal end at the site of the artery 3 between the cuff 1 and the body 2 until the limiter 20 contacts the cuff 1 end. By means of the unit 5 for varying static pressure, the body 2 area under the cuff 1 is compressed to an extent sufficient for arresting completely the circulation through the artery 3, whereafter by means of the unit 5 for varying static pressure, the value of pressure P_{M} of the cuff 1 to the body 2 area is smoothly decreased whereby the value of static pressure P_{T} is decreased. As soon as the value of static pressure P_{T} in the vicinity of the cuff 1 center has become less that the systolic arterial pressure Ps, the pulse waves 7 are generated. Upon detection of the first pulse wave by the detector 12, the unit 17 for processing the output signals by means of the static pressure sensor 14 measures the value of this pressure and records the measured value as the value of systolic arterial pressure Ps. The value of the cuff 1 pressure to the body 2 area is further decreased, while the unit 17 for processing the output signals continues to measure and record the static pressure upon detection of each consecutive pulse wave until disappearance of the pulse waves. The last measured value of the static pressure is recorded by the unit 17 for processing the output signals as the value of the diastolic arterial pressure Po. The cuff 1 pressure to the body 2 area is decreased to zero. The separating element 4 is removed from under the cuff, and readings of the unit 17 for processing the output signals are taken.

### INDUSTRIAL APPLICABILITY

The proposed method and device make it possible to accurately measure in semiautomatic or automatic mode two values of arterial pressure accepted in medical practice with guaranteed reliability sufficient for applying the method and device in the everyday medical practice virtually for all categories of patients.

## Claims

1. A method for measuring arterial pressure, wherein:
a pressure is produced, by means of a static pressure source, within a body area containing an artery;
the value of said static pressure is varied within a value range which necessarily includes a possible range of arterial pressure values;
the passage of pulse waves is detected;
the value of systolic and diastolic arterial pressure is determined based on said static pressure at a time of onset and disappearance of said pulse waves,
**characterized in that** said static pressure source is caused to hydraulically separate from a body area situated opposite thereto and containing an artery by placing a rigid separating element between the two and along the artery, said rigid separating element having a first side facing the body and a second side facing said static pressure source, said pulse waves are converted with the aid of said separating element into pressure waves which run along said rigid separating element on the first side thereof, and the passage of said pulse waves is detected with the aid of said pressure waves which run along the surface of said rigid separating element first side.

2. The method according to Claim 1, **characterized in that** the values of systolic and diastolic arterial pressure is determined based on the determined maximum and minimum values of said static pressure applied to said rigid separating element in the vicinity of the cuff center at a time of onset and disappearance of said pulse waves.

3. A device for measuring arterial pressure, comprising a static pressure source, a means for varying and measuring static pressure, a pulse wave detector, as well as a means for processing output signals, **characterized in that** said device comprises a rigid separating element which has a first contact surface for interacting with the body and a second contact surface arranged opposite thereto for interacting with said static pressure source, wherein inside said rigid separating element, said means for measuring static pressure is configured as a push-type pressure gauge with a contact area embedded into one of the two contact surfaces of said separating element, as well as said pulse wave detector whose sensing element is located on the first contact surface of said separating element

4. The device according to Claim 3, **characterized in that** said second contact surface of said rigid separating element is provided with a limiter for locking the position of said element under said static pressure source.

5. The device according to Claims 3 or 4, **characterized in that** said static pressure source is configured as a cuff.

6. The device according to Claims 3 or 4, **characterized in that** said sensing element of said pulse wave detector comprises a bandage embedded into said first contact surface of said rigid separating element by means of a hinge arranged along said bandage line of symmetry extending transversely to an artery.
